(19) Europäisches Patentamt
European Patent Office
Office européen des brevets

(11) **EP 4 295 776 A1**

(12) **EUROPEAN PATENT APPLICATION**

(43) Date of publication:
**27.12.2023 Bulletin 2023/52**

(21) Application number: **22180728.2**

(22) Date of filing: **23.06.2022**

(51) International Patent Classification (IPC):
***A61B 6/03*** (2006.01)    ***A61B 6/00*** (2006.01)
***G01T 1/20*** (2006.01)    ***G06T 11/00*** (2006.01)

(52) Cooperative Patent Classification (CPC):
**A61B 6/032; A61B 6/4208; A61B 6/481;**
**A61B 6/482; A61B 6/5205; A61B 6/5217;**
**G01T 1/36; G01V 5/0041; G06T 11/005;**
**G06T 2211/408**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **PROKSA, Roland**
  **Eindhoven (NL)**
• **KOEHLER, Thomas**
  **Eindhoven (NL)**
• **GRASS, Michael**
  **5656AG Eindhoven (NL)**

(74) Representative: **Philips Intellectual Property &**
**Standards**
**High Tech Campus 52**
**5656 AG Eindhoven (NL)**

(54) **SPECTRAL CT DECOMPOSITION TECHNIQUE**

(57)    A method for transforming projection data from a first set of spectral channels to a second, reduced, set of spectral channels. The selection of the second, reduced set of spectral channels may be based on parameters of the CT data acquisition or upon properties of the projection data, such as properties indicative of a degree of x-ray attenuation by the imaged body.

FIG. 3

EP 4 295 776 A1

**Description**

FIELD OF THE INVENTION

This invention relates to the field of spectral-CT imaging, and in particular to techniques associated with basis decomposition of spectral CT projection data.

BACKGROUND OF THE INVENTION

A sub-class of CT imaging is spectral CT imaging. This involves acquiring CT projection data at a number of different energy levels, and using this data to derive additional information about imaged structures, based for example on the known different spectral absorption characteristics of different materials. For example, this class of imaging can be used to obtain superior material differentiation in images, due to the differing energy dependence of x-ray attenuation on different tissues. In spectral CT imaging, there can be generated multiple different imaging datasets, each corresponding to a different component or channel of the spectral imaging, e.g. Compton Scatter and Photoelectric images, or images corresponding to different energy levels or bands of the x-ray spectrum, or images corresponding to different materials.

Multi-energy projection data is acquired, and this can be described as comprising multiple spectral channels of projection data, each spectral channel corresponding to a particular energy of x-ray radiation traversing the body. The different channels can be acquired by cyclically altering an energy level of the x-ray source (known as kVp switching) and/or can be acquired by emitting a broad band energy spectrum and using a detector which can detect radiation at multiple separable energy levels (such as a dual-layer integrating detector which simultaneously detects two spectrally distinct measurements using a front and a rear detector placed on top of one another, each configured to integrate over a different portion of the full x-ray spectrum of detected x-ray radiation).

It is also possible to use a combination of both of these techniques. In particular, QUAD CT is a spectral CT imaging technique recently developed which combines kVp-switching and dual layer detector technology. This effectively generates four spectral data channels corresponding to the following combinations: Low kVp detected in the inner (sometime also called the 'upper') detector layer (Li), Low kVp detected in the outer detector layer (Lo), High kVp detected in the inner detector layer (Hi) and High kVp detected in the outer detector layer (Ho). The inner (or upper) detector layer detects the lower energy band, the outer (or lower) detector layer detects the higher energy band.

A key step in the reconstruction for spectral CT is the basis decomposition, generally known as the material decomposition (MD). This maps the detected input projection data to effective line integrals of base materials or physical attenuation effects (e.g. photo-electric absorption and Compton scatter). For the ease of the following discussion, the basis decomposition is assumed to be photo-scatter decomposition.

The decomposed attenuation line integrals are typically subsequently reconstructed to material base images. Material decomposition can be interpreted as the inverse of the system forward model (FM). The FM is a set of equations describing the expected detector reading as a function of the unknown base material line integrals, the energy dependent attenuation of these materials and the spectral properties of the used scanner. Typically, no closed form can be found for the inverse of the FM. Most realizations of MD uses either numerical optimization techniques, neural network based methods or look-up table approaches. For dual-energy CT, MD is a 2x2 problem mapping two data channels to two base material integrals.

QUAD CT has four input channels and usually maps these four channels to two basis components/materials. Since the input data are noisy and there is in this case an overdetermined mapping, it is also possible to use statistical methods to search for the most likely solution of the FM given a measurement.

Statistical methods are computationally expensive. Given the large amount of input data, this poses a significant technical challenge for CT scanners. A potential solution to this problem is the use of a pre-computed look-up table (LUT). LUTs are pre-calculated solutions mapping the 4-dimensional (one dimension for each input channel) input data space to the material domain. If the input data are discretized with a reasonable resolution, the LUTs will become very large. The required size of the LUT is additionally increased by the fact that the FM is different for each detector pixel. This is because the spectral X-ray filtration differs for each pixel. Even a grouping of detector pixels with similar spectra cannot reduce the required LUT size to practical sizes. The actual LUT for the dual layer system uses 207 pixel groups and 203 LUT entries per channel resulting in a LUT with $8.5 \times 10^6$ points. The calculation time for one LUT is about 5 minutes for a standard PC. Keeping the same LUT resolution would require a LUT with $3.5 \times 10^{11}$ entries (which amounts to 1.3 TByte) and a calculation time of 140 days.

A second problem of MD is the so-called noise-induced-bias. The MD is a nonlinear function. If the input data contain noise, the (zero-mean) input noise may lose the zero-mean property for the output noise. This bias effect becomes larger for strong input noise (small SNR) and forms a real image bias problem for spectral imaging. The noise-induced-bias problem becomes more severe for QUAD CT because the X-Ray flux is split into four channels instead of two. An additional problem arises if the number of detected photons per reading becomes very small or even zero. This effect is called photon starvation and may cause streak-like artefacts.

Developments in the field of spectral CT decomposition, able to address one or more of the above problems, would be of value.

SUMMARY OF THE INVENTION

The invention is defined by the claims.

According to examples in accordance with an aspect of the invention, there is provided a computed tomography (CT) basis decomposition method, comprising:

obtaining first projection data comprising, for each of an array of detector pixels, data for each of a first set of three or more spectral channels,

converting the first projection data to second projection data, wherein the second projection data comprises, for each of the array of detector pixels, data for each of a second set of two or more spectral channels, the second set of spectral channels including at least one virtual spectral channel formed from a combination of two or more of the first set of spectral channels, and wherein the second set comprises fewer channels than the first set;

applying a basis decomposition procedure to the second projection data to derive basis component data for each of at least two basis components.

Preferably, the combination used in the conversion to the second set of spectral channels is determined based on received information about one or more imaging parameters during acquisition of the first projection data, and/or is determined based on analysis of at least a portion of the first projection data.

Embodiments of the invention are based on the concept of reducing the total number of spectral channels of the source projection data (acquired from the detector in the CT scanning apparatus) to a smaller number, whereby basis component decomposition (e.g. material basis decomposition) can be performed with greater simplicity, due to the smaller number of spectral channels. Rather than simply apply a static conversion process, in which the whole of the first projection dataset is converted into equivalent data for the same fixed set of second spectral channels, the inventors have proposed to determine the particular configuration or composition of the second set of spectral channels in dependence upon parameters of properties of the projection data or of the acquisition apparatus during acquisition of the projection data. The second set of spectral channels can vary between pixels of the data in some embodiments, and/or between frames of data (where each frame corresponds to a particular projection at a particular angular and axial position of the gantry, and includes data for the whole array of detector elements/pixels). This dynamic configuration of the spectral channel reduction operation improves quality of the resulting decomposed data.

In some embodiments, the method may be executed separately for the data of each respective detector pixel.

In some embodiments, the combination used in the conversion to the second set of spectral channels is different for the data of at least two different pixels of the detector pixels. In other words, the particular second set (combination) of spectral channels can vary for the different detector pixels.

In some embodiments, the second set of spectral channels includes, either singly, or in combination as a virtual spectral channel, all of the first set of spectral channels.

In some embodiments, at least the conversion step is performed separately for the data of each of the different detector pixels.

In some embodiments, each virtual spectral channel is formed from a sum of a selection of the first spectral channels. In other words, a 'combination' means a sum in this case.

In some embodiments, the second set of spectral channels is a set of two spectral channels. In some embodiments, the basis decomposition procedure derives basis component data for a set of two basis components. If applied in combination, these two features reduce the decomposition problem to 2x2 decomposition (2 spectral channels, 2 basis components).

In some embodiments, the first spectral CT projection data is data from a dual-layer integrating detector comprising an inner detection layer and outer detection layer. Additionally or alternatively, the data may correspond to data from a dual-energy kvp-switching CT scan protocol in which x-ray radiation at two or more different energies is intermittently projected through the examination region (and scanned object) toward the detector. Where the two are used together, this corresponds to the QUAD imaging protocol discussed above. In this case, each detection layer records measurement data for each of two emitted x-ray energies.

The first spectral CT projection data may in this case comprise data for four spectral channels, each corresponding to a respective combination of one of the said detection layers of the detector and one of the energy levels of the kVp-switching protocol.

For ease of later reference, these four spectral channels can be explicitly recited and labelled as corresponding to the following combinations:

> lower kVp energy emission, detected in the inner detection layer (Li);
> lower kVp energy emission, detected in the outer detection layer (Lo);
> higher kVp energy emission, detected in the inner detection layer (Hi); and
> higher kVp energy emission detected in the outer detection layer (Ho).

In some embodiments, the second set of spectral channels consists of a set of two spectral channels.

In some embodiments, the second set of spectral channels consists of one of the following combinations of two spectral channels:

> Li, and a virtual spectral channel comprising a combination of Hi, Lo and Ho;
> Hi, and a virtual spectral channel comprising a combination of Li, Lo and Ho;
> Lo, and a virtual spectral channel comprising a combination of Li, Hi and Ho;
> Ho, and a virtual spectral channel comprising a combination of Li, Hi, and Lo;
> a virtual spectral channel comprising a combination of Li and Hi, and a virtual spectral channel comprising a combination of Lo and Ho;
> a virtual spectral channel comprising a combination of Li and Lo, and a virtual spectral channel comprising a combination of Hi and Ho; or
> a virtual spectral channel comprising a combination of Li and Ho, and a virtual spectral channel comprising a combination of Hi and Lo.

In some embodiments, the set of second spectral channels into which the first projection data is converted is determined individually for each pixel of data. A pixel of data means the first projection data for a single one of the detector pixels. By selecting the composition of the second set of spectral channels separately for each pixel of data, quality of resulting data is improved.

In some embodiments, the second set of spectral channels for each pixel of data is determined based on one or more acquisition parameters or settings of a CT imaging apparatus used to acquire at least the respective pixel of the first projection data.

In some embodiments, the determining of the second set of spectral channels for each pixel of data is determined based one or more acquisition parameters or settings of a CT imaging apparatus used to acquire the first projection data which vary as a function of time over the course of acquiring a first projection data set. These might include for example, a scanner gantry rotational position, or a patient axial position relative to the scanner gantry, or radiation strength.

In some embodiments, the determining of the second set of spectral channels for each pixel of data is based on radiation strength of the radiation emitted in acquiring at least the respective pixel of the first projection data. Different combinations of second spectral channels result in greater or lesser SNR in decomposed data depending upon the radiation strength, as will be illustrated later. This relationship can be computed and stored in advance for use during execution of the data processing method. Thus, varying the second set of spectral channels based on the radiation strength improves overall SNR of decomposed data.

In some embodiments, the scan protocol is a dose modulation protocol, wherein the radiation strength of the emitted radiation is varied as a function of time over the course of acquiring a first projection data set. Thus, here the particular set of second spectral channels may vary between pixels and/or may vary between frames of data (different frames corresponding to different time points throughout the scan, and for example different angular and axial positions of the gantry relative to the examination region).

To facilitate this, there may be a stored record of radiation strength as a function of time for the scan which the method may access, or the method may comprise receiving a live indication of radiation strength as a function of time, so that the radiation strength corresponding to each frame of data can be known.

In some embodiments, the determining of the second set of spectral channels for each pixel of data is determined based on an effective basis component path length through the object associated with the projection data for the given pixel. Different combinations of second spectral channels result in greater or lesser SNR in decomposed data depending upon the equivalent material or basis component path length through the object for each pixel of data, as will be illustrated later. Thus, varying the second set of spectral channels based on this factor improves overall SNR of decomposed data. This is thus an example of determining the second set of spectral channels based on analysis of at least a portion of the first projection data (mentioned above).

In some embodiments, the method comprises: converting the first projection data into each of a plurality of candidate sets of second spectral channels to derive a plurality of candidate second projection data sets; applying a decomposition procedure to each of the candidate second projection data sets to obtain a plurality of candidate decomposed second projection data sets; obtaining a variable or parameter associated with each candidate decomposed second projection dataset, either from processing of the decomposed data set or from the decomposition procedure. The method may then further comprise either: selecting one of the candidate second projection datasets for use in forming a final decomposed dataset based on the respective values of said variable or parameter, or, forming a weighted combination of at least a subset of the candidate second projection datasets for use in forming a final decomposed dataset using the said variables or parameters in setting the weighting factors.

In some embodiments, said variable or parameter of each candidate decomposed dataset is a computed signal-to-noise ratio (SNR) estimate associated with the respective candidate decomposed data set. Thus in this case, the converting the first projection data to the second projection data may be performed multiple times, to form multiple different candidate second sets of spectral channels (as stated above), and wherein the basis decomposition procedure is applied to each candidate second set of spectral channels, and wherein an SNR is determined for each decomposed candidate second set of spectral channels, and wherein the candidate second set of spectral channels with the highest SNR is selected for use in forming an image.

In some embodiments, the decomposition procedure comprises use of a respective lookup table to map each candidate second projection data set to a candidate decomposed second projection data set, and wherein each record of each lookup table corresponding to a particular set of input data values includes a set of decomposed data values and includes said variable or parameter. Thus in this case, the basis decomposition may be performed using a precomputed look-up table (LUT) and wherein the LUT maps values of a particular second set of spectral channels to corresponding basis component effective path lengths, and wherein, for each LUT entry, a pre-computed data item is stored in addition to the basis component path lengths for use in controlling a selection from multiple sets of second spectral channels. The data item can either be a discrete selector to identify the basis decomposition result to use or it can be a set of weighting factors for a weighted addition of multiple basis decomposition results.

In some embodiments, and as will be explained later, the determining the second set of spectral channels into which the first projection data is converted for a given pixel comprises computing, for at least a first candidate set of second spectral channels, a ratio of data values of at least two of the first spectral channels for the pixel, and selecting the candidate set to be the second set of spectral channels only if the ratio meets a pre-defined threshold.

This process may of course be repeated if the ratio does not meet the threshold, for different candidate sets of second spectral channels, until a candidate set is found which meets the threshold.

In some embodiments, the set of basis components of the basis component data is the same for all pixels of the first projection data.

In some embodiments, the method comprises applying a blending operation to the basis component data such as to smooth a transition between basis component data of at least a first pixel and basis component data of at least a second pixel, where the data for the respective pixels was decomposed using different respective second sets of spectral channels.

The blending operation may comprise performing a search for such transitions within the data for a given projection across detector rows or columns. In a neighborhood of pre-defined size and shape close to these identified transitions, a smooth ramp function may be applied for the blending across the transition.

Embodiments of this invention can be applied not only to traditional full-rotation CT scan data (including cone beam CT), but also to CT projection data acquired from a C-arm system, for example having a dual layer detector and kVp switching. The method can also be applied for 2D projection processing, where the latter refers to a C-arm type system in which 2D images are acquired directly from the detector. This is in contrast to CT imaging in which multiple detector readings (at different gantry angles) are reconstructed to (most frequently) form 3D image data.

Another aspect of the invention provides a computer program product comprising computer program code configured, when executed by a processor, to perform a method in accordance with any embodiment or example in this disclosure, or in accordance with any claim. The processor may in examples be operatively coupled to a CT scanning apparatus for receiving the first projection data in real time. The processor may in other examples be operatively coupled to a data store storing a recording of CT projection data.

Another aspect of the invention provides a processing device, comprising: an input/output; and one or more processors, adapted to perform the following steps:

receive at the input/output first projection data comprising, for each of an array of detector pixels/elements, data for each of a first set of three or more spectral channels;
convert the first projection data to second projection data, wherein the second projection data comprises, for each of the array of detector pixels, data for each of a second set of two or more spectral channels, the second set of spectral channels including at least one virtual spectral channel formed from a combination of two or more of the first set of spectral channels, and wherein the second set comprises fewer channels than the first set; and
apply a basis decomposition procedure to the second projection data to derive basis component data for each of at least two basis components.

Preferably, the second set of spectral channels includes, either singly, or in combination as a virtual spectral channel, all of the first set of spectral channels;

Preferably, the second set of spectral channels is determined based on received information about one or more imaging parameters during acquisition of the first projection data, and/or is determined based on analysis of at least a portion of the first imaging data.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a block diagram of steps of a method in accordance with one or more embodiments of the invention;
Fig. 2 shows a block diagram of an example system in accordance with one or more embodiments of the invention;
Fig. 3 and Fig. 4 schematically illustrate example projection data conversion operations in accordance with one or more embodiments, for converting from a first (source) set of spectral channels to a second set of spectral channels;
Fig. 5 and Fig. 6 illustrate a relationship between a SNR of decomposed data and two respective scan or imaging parameters which may vary over time and/or for different pixels of data;
Fig. 7 illustrates an example selection procedure for selecting a second set of spectral channels for a given first projection data set;
Fig. 8 illustrates an example procedure for forming a weighted combination of the decomposed values for two candidate second sets of spectral channels; and
Fig. 9 shows an example CT imaging apparatus.

DETAILED DESCRIPTION OF THE EMBODIMENTS

The invention will be described with reference to the Figures.

The invention provides a method for transforming projection data from a first set of spectral channels to a second, reduced, set of spectral channels. The selection of the second, reduced set of spectral channels may be based on parameters of the CT data acquisition or upon properties of the projection data, such as properties indicative of a degree of x-ray attenuation by the imaged body or of radiation strength of emitted x-ray radiation.

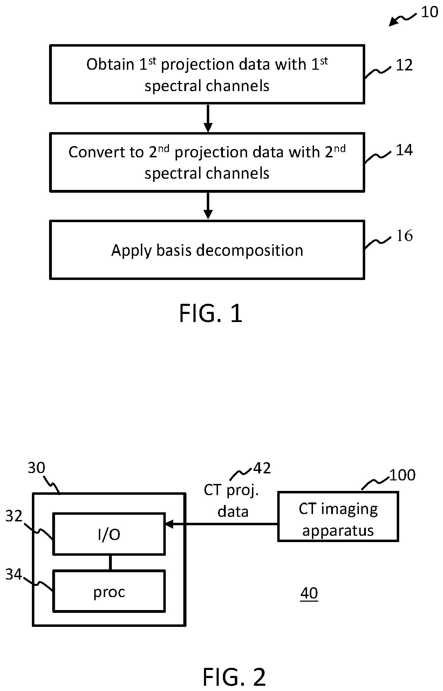

Fig. 1 outlines in block diagram form steps of an example method according to one or more embodiments. The steps will be recited in summary, before being explained further in the form of example embodiments.

The method 10 comprises a step of obtaining 12 first projection data comprising, for each of an array of detector pixels/elements, data for each of a first set of three or more spectral channels. The array of detector pixels forms a grid for example.

The method 10 comprises converting 14 the first projection data to second projection data. The second projection data comprises, for each of the array of detector pixels, data for each of a second set of two or more spectral channels, the second set of spectral channels including at least one virtual spectral channel formed from a combination of two or more of the first set of spectral channels, and wherein the second set comprises fewer channels than the first set. In this context, a combination of first spectral channels means for example a sum of the first spectral channels, which means, for a given pixel, a sum of the recorded data values for the given pixel for the combined first spectral channels. The sum may be a weighted sum in some cases.

The method 10 further comprises applying a basis decomposition procedure to the second projection data to derive basis component data for each of at least two basis components. The basis decomposition procedure may be a material basis decomposition procedure, e.g. water, iodine, calcium. The basis decomposition procedure may decompose data into components representing different mechanisms of x-ray attenuation, such as photoelectric effect and Compton scatter.

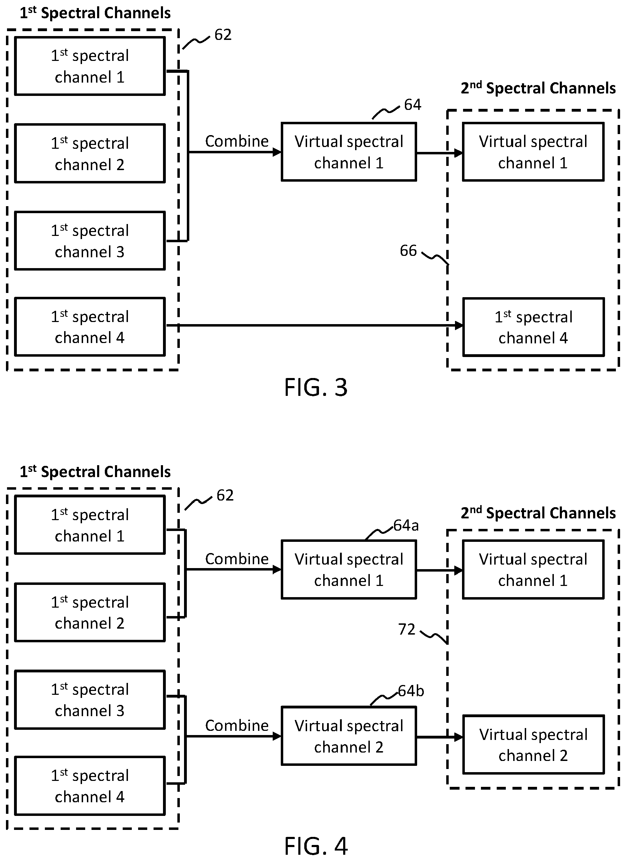

The second set of spectral channels preferably includes, either singly, or in combination as a virtual spectral channel, all of the first set of spectral channels. This will be illustrated later with reference to Fig. 3 and Fig. 4.

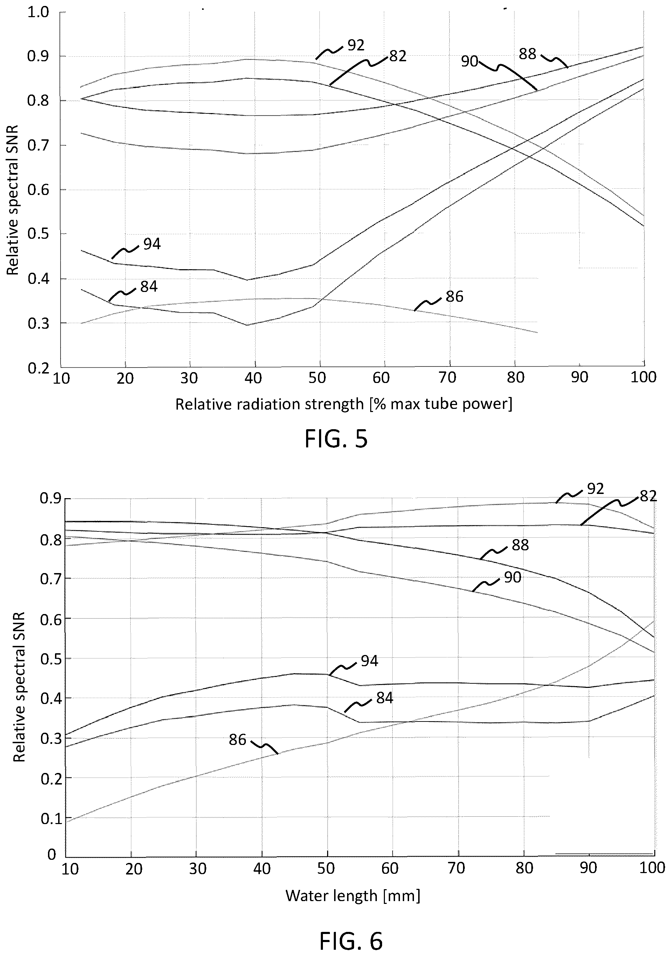

The second set of spectral channels is determined based on received information about one or more imaging parameters during acquisition of the first projection data, and/or is determined based on analysis of at least a portion of the first projection data. This will be illustrated later with reference to Fig. 5 and Fig. 6.

With regards to the basis decomposition procedure, the skilled person will be aware of numerous different methods available in the art for performing this, which are suited for different numbers of input spectral channels, and for decomposing spectral data into different sets of output basis components, e.g. Compton Scatter and Photoelectric effect components, water and iodine components, calcium, bone content and so on.

Fig. 2 illustrates in block diagram form components of an example system 40 in accordance with an aspect of the invention. The system comprises a processing device 30 comprising an input/output 32 and one or more processors 34 configured to perform the method in accordance with any of the examples or embodiments in this disclosure. The processing device can be provided by itself as a separate aspect of the invention. The input/output 32 of the processing device 30 may be operatively coupled to a CT imaging apparatus 100 in some examples, for receiving the first projection data 42. This may be received in real time. The data may be cached the apparatus for the whole scan and then transferred to the processing device 30 all at once at the end of the scan. In other examples, the data can be received at the input/output from a separate datastore which has a record of previously obtained data. The whole system 40 shown in Fig. 2 may be provided as another aspect of the invention.

An example implementation of the method according to a particular set of embodiments will now be described by way of illustration of the above-summarized concept of the invention. It will be appreciated that not all features of this particular set of embodiments are essential to the inventive concept, and are described to aid understanding and to provide an example to illustrate the inventive concepts.

In accordance with this particular set of embodiments, virtual spectral channels (VSCs) are utilized for reducing a four-channel input set of first projection data to a 2-channel set of second projection data. Furthermore, in this set of embodiments, the 2-channel set of second projection data is decomposed with a decomposition algorithm which results in two basis components. This, it may be said that this set of embodiments reduces a 4x2 decomposition problem to a 2x2 decomposition problem, where the first number represents the number of spectral channels in the projection data and the second number represents the number of basis components into which the projection data is to be decomposed. Decomposing four spectral channels into two basis components is a difficult and resource intensive process. Reduction to a 2x2 decomposition problem streamlines the procedure.

In accordance with this particular set of embodiments, the input first projection data is assumed to be data acquired using a QUAD CT imaging protocol previously described. In QUAD-CT imaging, the first spectral CT projection data is data from a dual-layer integrating detector comprising an inner detection layer and outer detection layer, and furthermore corresponds to data from a dual-energy kvp-switching CT scan protocol in which x-ray radiation at two or more different energies is intermittently projected toward the detector. Thus, each detection layer records measurement data for each of two emitted x-ray energies. This results in first spectral CT projection data comprises data for four spectral channels, each corresponding to a respective combination of one of the said detection layers of the detector and one of the energy levels of the kvp-switching protocol.

By way of ease of later reference, these four first spectral channels can be listed in terms of the particular combination of detector layer and kvp energy which they represent, as follows:

Lower kVp energy emission, detected in the inner detection layer (this spectral channel will be referred to as "Li");
Lower kVp energy emission, detected in the outer detection layer (this spectral channel will be referred to as "Lo");
Higher kVp energy emission, detected in the inner detection layer (this spectral channel will be referred to as "Hi"); and
Higher kVp energy emission detected in the outer detection layer (this spectral channel will be referred to as "Ho").

To facilitate the conversion 14 from a four-channel first projection dataset to a two-channel second projection dataset, a set of one or more virtual spectral channels can be defined which each represent a combination of two or more of the first spectral channels. A combination in this example means a sum or weighted sum.

By way of example, the virtual spectral channels used as members of the second set of spectral channels for any one or more pixels of data might include any one or more of the following:

"HiLoHo": a combination of Hi, Lo and Ho first spectral channels;
"LiLoHo": a combination of Li, Lo and Ho first spectral channels;
"LiHiHo": a combination of Li, Hi and Ho first spectral channels;
"LiHiLo": a combination of Li, Hi and Lo first spectral channels;
"LiHi": a combination of Li and Hi first spectral channels;
"LoHo": a combination of Lo and Ho first spectral channels;
"LiLo": a combination of Li and Lo first spectral channels;
"HiHo": a combination of Hi and Ho first spectral channels;
"LiHo": a combination of Li and Ho first spectral channels;
"HiLo": a combination of Hi and Lo first spectral channels.

By way of illustration, the inventors have found that there are seven most useful sets of second spectral channels, each consisting of at least one of the above virtual spectral channels, in combination either with one of the first spectral channels or in combination with another of the above virtual spectral channels. Using the naming convention above, these particularly useful sets of second spectral channels are the pairs as follows: Li and HiLoHo, Hi and LiLoHo, Lo and LiHiHo, Ho and LiHiLo, LiHi and LoHo, LiLo and HiHo and LiHo and HiLo.

Thus it can be seen that the second set of spectral channels can be formed from any of a range of permutations of virtual spectral channels, and optionally first spectral channels.

The forward model (FM) for a QUAD CT pixel reading using an two-layer energy integrating detector is:

$$d_{l,v,p} = \int dE\, E\, T_v(E)\, D_l(E) F_p(E) e^{-A\mu_P(E) - B\mu_S(E)}$$

with E being the photon energy, $T_v$ being the effective tube output spectrum for the low and high energies of the kVp switching protocol, $D_l$ being the detector signal response, $F_p$ being the effective beam filtration for pixel p, *A, B* being the effective line integrals for photo-electric attenuation and scatter attenuation (i.e. the two basis components in this example), $\mu_P(E)$ and $\mu_S(E)$ being the linear attenuation of photo-electric attenuation and scatter attenuation, $l \in \{i, o\}$ being the detector layer index, $v \in \{L, H\}$ being the kVp index and *p* being the pixel index.

A virtual spectral channel detector signal is in this example the sum of one or more of the source (first) spectral channels:

$$d_{p,X} = \sum_{v,p \in X} d_{p,v,p}$$

The related forward model (FM) for this example spectral channel (for one particular detector layer) becomes:

$$d_{p,X} = \int dE\, E \left( \sum_{v,p \in X} T_v(E) D_l(E) \right) F_p(E) e^{-A\mu_P(E) - B\mu_S(E)}$$

with Xbeing a set a tuples (v,p) defining the selected detector layer and pixel. The reduction of the QUAD 4x2 basis decomposition to a 2x2 decomposition problem removes the technical challenges of higher order basis decompositions.

The concept is illustrated further in Fig. 3 and Fig. 4.

Fig. 3 illustrates an example conversion 14 process from first projection data comprising measurement values for each detector pixel for each of a first set 62 of spectral channels, into second projection data comprising second measurement values for each detector pixel for each of a second set 66 of spectral channels. As illustrated, the second set 66 of spectral channels in this case has two spectral channels, while the first set 62 has four. The second set of spectral channels comprises one virtual spectral channel 64 (virtual spectral channel 1) which is constructed as combination (in this case, a sum) of a subset of the set of first spectral channels 62, in this example a subset of three of the first spectral channels (1st channel 1, 1st channel 2 and 1st channel 3).The second set of spectral channels is formed from the virtual spectral channel 1, and the remaining 1st spectral channel (1st spectral channel 4) which does not form any part of the virtual spectral channel 1.

Fig. 4 shows a further example. In this case, the second set of spectral channels 72 is composed of two virtual spectral channels 64a, 64b (virtual spectral channel 1 and virtual spectral channel 2). Each of these virtual spectral channels is formed from a respective combination of two of the first set 62 of spectral channels. Thus, in this case, the second set of spectral channels comprises none of the original first spectral channels, unlike in the example of Fig. 3.

As is therefore apparent from Fig. 3 and Fig. 4, the second set of spectral channels includes at least one virtual spectral channel, and may include more than one virtual spectral channel, and may include, in combination with at least one spectral channel, one or more of the (unaltered) first spectral channels.

As illustrated in the example of Fig. 3 and Fig. 4, it is preferable if the second set of spectral channels includes, either singly, or in combination as a virtual spectral channel, all of the first set of spectral channels. For example, in the case of Fig. 3, the second spectral channels comprise the first spectral channel 4 singly and comprises the first spectral channels 1, 2 and 3 in combination in the form of virtual spectral channel 1. Thus, the set of (in this case two) second spectral channels includes all of the first spectral channels, either singly, or as part of a combination forming a constructed virtual spectral channel. Similarly, in Fig. 4, the second spectral channels comprise first spectral channels 1 and 2 in combination in the form of virtual spectral channel 1, and comprise first spectral channels 3 and 4 together in the form of virtual spectral channel 2.

It is to be noted that instead of four spectral channels, the first set of spectral channels can comprise any number of three of more spectral channels.

It is to be noted that instead of two spectral channels, the second set of spectral channels can comprise any number of two or more spectral channels, so long as the number of second spectral channels is smaller than the number of first spectral channels.

A part of the method is the determining of the composition of the second set of spectral channels into which the first set of projection data (and the first spectral channels) are transformed. As noted above, this can vary for data of different detector pixels in preferred examples, and/or for different frames of data corresponding to different time points throughout the scan. More generally, the second set of spectral channels is preferably determined based on properties of the specific set of first projection data which is being processed, and/or upon parameters of the specific data acquisition operation used to acquire the first projection data.

Configuration of the second set of spectral channels in accordance with one particular set of embodiments will now be described.

The performance of a selected set of second spectral channels - in terms of sensitivity to noise-induced-bias, photon starvation effect and the spectral separation power - depends on parameters/settings of the scan protocol used to acquire the projection data, and upon physical properties of the scanned object.

In accordance with this set of embodiments, a key idea is to dynamically select the second spectral channel set based on one or more of the scan parameters and/or based on properties of the (scanned) object attenuation on a pixel reading by pixel reading basis. For each individual pixel reading, the optimal second spectral channel set selection is used for the basis decomposition.

A useful optimization criterion for the selection process is the spectral separation power. In a simulation study, the inventors simulated a QUAD CT system with a two-layer integrating detector and a kVp-switching protocol. The inventors estimated the detection signals for scan protocols with different scan parameter settings and different object thicknesses. The inventors uses the expected signal-to-noise ratio (SNR) of a virtual-non-contrast-image (VNC) obtained from a Water-Iodine material decomposition (a process which will be well-known to a skilled reader) and named this quantity 'spectral SNR'. The inventors performed this for all possible second spectral channel set selections and compared the results relative to the result from a standard decomposition of 4-channel QUAD data into the same two basis components (water/iodine), i.e. a 4x2 decomposition.

Fig. 5 shows the spectral SNR obtained for each of the example seven pairs of second spectral channels discussed above as a function of a radiation strength used in the scan protocol to obtain the data.

Fig. 6 shows the spectral SNR obtained for each of the example seven pairs of second spectral channels discussed above as a function of the object thickness (in this simulation, this was obtained from a water phantom with different thicknesses).

In each graph, the line labels correspond to the second spectral channel sets as follows:

| Line 82: | Li and HiLoHo; | Line 84: | Hi and LiLoHo; |
| Line 86: | Lo and LiHiHo; | Line 88: | Ho and LiHiLo; |
| Line 90: | LiHi and LoHo; | Line 92: | LiLo and HiHo; |
| Line 94: | LiHo and HiLo. | | |

The results show that optimal selection of the second spectral channel set (meaning that which yields the greatest SNR in the decomposed data) varies with the parameters of the scan protocol (in this example, radiation strength), and as a function of object attenuation. Optimal results can clearly be obtained with a dynamic selection. It is to be noted that even within a single projection, pixels with different object attenuation can have different selections. The optimal protocol-related second spectral channel set selection may also change dynamically during a scan, for example in the case of a dynamic dose modulation (DOM) scan protocol. In a dose modulation protocol, the radiation strength of the emitted radiation is varied as a function of time over the course of acquiring a first projection data set. If the tube current or the duty cycle of the kVp-cycle changes, the optimal second spectral channel set selection may change accordingly and may be dynamically adjusted.

Thus, as a more general principle, it can be seen that the determining of the second set of spectral channels for each pixel of data may be determined based on a radiation strength of the radiation emitted in acquiring at least the respective pixel of the first projection data. It can also be seen that, additionally or alternatively, determining of the second set of spectral channels for each pixel of data may be determined based on an effective basis component path length through the object associated with the projection data for the given pixel. The latter principle is observable from the relationship in Fig. 6 which shows that the SNR varies as a function of a single-material object thickness. Even more generally, it can be seen that determining of the second set of spectral channels for each pixel of data may be determined based on a magnitude of x-ray attenuation associated with at least the projection data for the respective detector pixel.

In practice, the radiation strength corresponding to a particular frame of data and/or a particular pixel within that frame may be obtained from a record which was generated during the scan, or another source of this information, e.g. scan protocol information stored in association with the first projection data. In practice, the object thickness or basis component effective path length, or degree of attenuation corresponding to a particular pixel can be derived based on performing an initial basis decomposition, reviewing the resulting path length, and then iteratively adjusting the selection of the second set of spectral channels if a different set would yield a higher SNR, given the path length.

The particular relationship or mapping between radiation strength and/or object attenuation and the optimal set of second spectral channels can be pre-computed in advance and stored in the form of a lookup table or transfer function, accessible during execution of the method (e.g. stored in a memory).

Another, more direct, way to achieve the same result is simply to perform an initial basis decomposition, directly evaluate the SNR, and then adjust the selected set of second spectral channels if a higher SNR is possible. In some examples, multiple different candidate second sets of spectral channels may be formed for the data of a given pixel, and wherein each is processed with the basis decomposition, and where the SNR of the results are compared to identify and select the candidate set of second spectral channels which yields the highest SNR.

The above results also show that for given boundary conditions (e.g. a certain detector response and certain ramp-characteristics for the x-ray tube), only two relevant combinations of second spectral channels are needed, which reduces the implementation effort.

In particular, in the low-dose regime (i.e. low radiation strength), sufficient dose (for image quality) is provided at the low-kV channel and best performance is achieved by a set of two virtual spectral channels which are each formed from a combination of the two layers at one of the kVp energies. In other words, in this case, optimal performance is achieved with a second set of spectral channels consisting of LiLo and HiHo. It will be appreciated that in this case, the resulting second set of spectral channels effectively corresponds to a set of two spectral channels that would be yielded from a simple dual-energy kVp regime using a single layer detector (since the channels for the two layers are combined together). Thus the reconstruction reduces effectively to a standard kVp-switching model.

On the other hand, for the high-dose case (i.e. high radiation strength), where the desired dose can only be reached by reducing the radiation flux at low kV, it becomes beneficial to increase the flux for the low energy channel by combining the Li channel not only with the Lo channel but also with the Hi channel (see Fig. 5). To explain further, for high dose protocols, it is necessary to reduce the flux for low energy photons. Typically, this means changing the low kV-high kV duty cycle such that there is greater amount of high energy flux. In these cases, the SNR for lower energies becomes poor and thus better results are achieved if the Hi channel is added to the low energy selection.

Instead of using an integrating detector, it is to be noted that the inventive concept is also applicable for projection data acquired using a multi-channel photon-counting detector. In this case, the first set of spectral channels corresponds to the readings of the energy bins of the detector, and optionally the high and low kvp energy level (if kVp switching is used in combination with the photon count detector). In this case, by way of example, the variable number of detector energy bins may be combined to form one higher energy and one lower energy bin.

It is noted that embodiments of this invention can be applied not only to traditional full-rotation CT scan data (including cone beam CT), but also to projection data acquired from a C-arm system, for example having a dual layer detector and kVp switching.

Although the principles of certain embodiments of the invention have been described above in terms of a particular configuration (dual layer detector, kvp-switching), it will of course be recognized that the inventive concept has more general application.

For example, the method according to the invention could be described as a spectral channel reduction method to reduce the basis component decomposition from an *AxB* mapping to a *CxB* with *C<A*. Here, A corresponds to the number of spectral channels in the source data (the first spectral channels), C corresponds to the number of spectral channels after conversion (i.e. the second set of spectral channels) and B corresponds to the number of basis components into which the spectral data is being decomposed.

As noted above, the optimal selection of the second set of spectral channels depends on several protocol and patient absorption factors. It is proposed in a preferred set of embodiments to use a data-driven automatic selection of the second set of spectral channels to simplify the implementation. By way of example, a simple selection criterion may be based on analysis of the first projection data itself, for instance per pixel reading, or similar semi empirical rules.

By way of one example, a rule may be devised in advance which is based on relative values (e.g. a ratio) of two or more candidate spectral channels, and wherein a threshold is set for the ratio. In other words, in some embodiments, determining the second set of spectral channels into which the first projection data is converted for a given pixel may comprise computing, for at least a first candidate set of second spectral channels, a ratio of data values of at least two of the first spectral channels for the pixel, and selecting the candidate set to be the second set of spectral channels only if the ratio meets a pre-defined threshold. By way of one example, considering again the QUAD scanning protocol already discussed above, a rule might be pre-defined wherein if the sum of LiLo relative to the sum HiHo is below a certain threshold, there may be assumed an SNR mismatch for the LiLo and HiHo selection, and the selection of the second set of spectral channels may be switched to LiLoHi and Ho, i.e.

$$selection = \begin{cases} LiLoHi \text{ and } Ho & \dfrac{d_{L,i} + d_{L,o}}{d_{H,i} + d_{H,o}} < x \\ LiLo \text{ and } HiHo & otherwise \end{cases}$$

where x is a threshold.

This heuristic method is based on the insight that the SNR of a basis decomposition is typically optimal if the SNR of the input channels are similar. In other words, a strong misbalance of the input channel SNRs is suboptimal. Furthermore, the signal strength of a channel is a reasonable indication of the SNR of the signal. Taking these facts together, it may be expected that the ratio of the added signals would be close to 1, if the SNR of the input channels are similar. If the misbalance (ratio) of the SNR's reaches a pre-defined threshold (x), the channel selection may be changed, e.g. iteratively, to approach close to 1.

In a typical case, patient absorption is maximal somewhere in the central region of a projection (i.e. central region of the detector array) and decreases towards the periphery. This may lead to the occurrence of a discrete or step change (spatially across the pixel array) in the chosen set of second spectral channels in many projections. Even small imperfections in the basis decomposition may cause streak artefacts as a result of such discrete transitions of the selection of second spectral channels which are used for the basis decomposition. In some embodiments, this potential problem may be reduced with a smooth blending of the transition. For example, a smoothly variable blending factor $0 \le b \le 1$ may be used to apply a smooth transition between decomposed pixels corresponding to one set of spectral channels and those corresponding to another within a same projection by combining the basis decomposition results with a weighted summation of the two pixel sets.

Thus for example the method comprises applying a blending operation to the basis component data such as to smooth a transition between basis component data of at least a first pixel and basis component data of at least a second pixel, decomposed using different respective second sets of spectral channels.

For example, a blending operation may comprise searching for spectral channel selection transitions within a projection in detector rows. In a certain neighborhood close to these transitions, a smooth ramp may be applied for blending between two regions for which different sets of second spectral channels have been used.

In some examples, the numerical result of a data driven selection of the second set of spectral channels (as explained above) may be used to generate a blending factor close to transitions.

In some embodiments, the basis decomposition may be performed using a precomputed look-up table (LUT) and wherein the LUT maps values of a particular second set of spectral channels to corresponding basis component effective path lengths, and wherein, for each LUT entry, a pre-computed data item is stored in addition to the basis component path lengths for use in controlling a selection from multiple sets of second spectral channels. The data item can either be a discrete selector to identify the basis decomposition result to use or it can be a set of weighting factors for a weighted addition of multiple basis decomposition results.

To explain further, there may be a respective lookup table (LUT) for each possible second set of spectral channels (or for each of a pre-defined group of second sets of spectral channels), and wherein each respective lookup table is used to perform the basis decomposition procedure for a projection dataset which has been converted to that particular set of second spectral channels. In at least one of the lookup tables, for each entry corresponding to a particular set of values for the second spectral channels, there may also be listed a variable or parameter which is for use in selecting from multiple candidate sets of second spectral channels, to choose a favored one. For example, this variable might correlate with SNR of the resulting decomposed value set which is the output from the LUT. A rule may be set whereby if this value in a given lookup table is above a certain threshold, the candidate decomposed data from this given table is used, and if lower than the threshold, then the candidate decomposed data from the other lookup table is used. Alternatively, the pre-computed variables or data items corresponding to the decomposed data sets might be directly compared, and wherein that decomposed dataset with the highest variable is selected for use.

Alternatively, the variable or data item might be used as a weighting factor for forming a weighted combination of the decomposed values of the multiple candidate sets of second spectral channels.

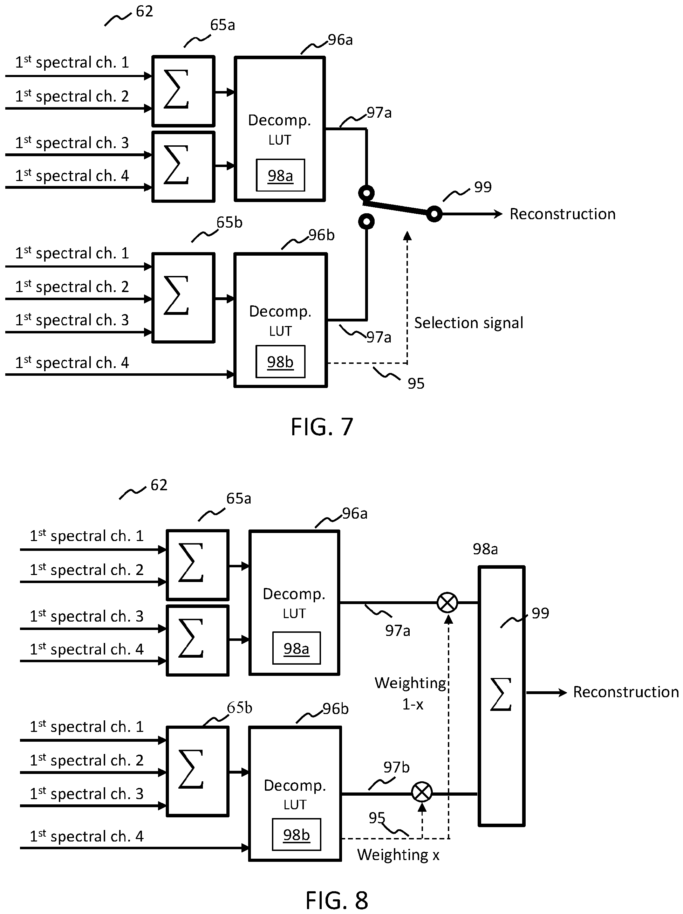

The above is illustrated schematically in Fig. 7 and Fig. 8. Fig. 7 shows the option in which a single final decomposed dataset is used. Fig. 8 shows the option in which a there is formed a weighted combination of the multiple candidate decomposed datasets.

As shown, in these embodiments, the method comprises converting the first projection data set, comprised of data for each of a first set of spectral channels 62 into each of a plurality of candidate sets 65a, 65b of second spectral channels to derive a plurality of candidate second projection data sets. In the illustrated example, one candidate set 65a of second spectral channels is formed from two virtual spectral channels, each formed of a sum of two of the first 62 spectral channels. The other candidate set 65b of second spectral channels is formed from one virtual spectral channel (formed from a sum of three of the first spectral channels 62) and one of the first spectral channels.

The method comprises applying a respective decomposition procedure 96a, 96b to each of the candidate second projection data sets (i.e. the data of second spectral channels) to obtain a plurality of candidate decomposed second projection data sets. In the illustrated example, the decomposition procedure comprises use of a respective lookup table (LUT) 96a, 96b to map each candidate second projection data set 65a, 65b to a candidate decomposed second projection data set 97a, 97b.

The method further comprises obtaining a data item, e.g. a variable or parameter 98a, 98b associated with each candidate decomposed second projection dataset 97a, 97b, either from processing of the decomposed data set or from the decomposition procedure. In the illustrated example, it is obtained from the decomposition procedure. In particular, it is obtained from the lookup tables 96a, 96b. In particular, a respective record of each lookup table corresponding to a particular set of input data values 65a, 65b includes a set of decomposed data values 97a, 97b and includes said variable or parameter 98a, 98b. The variable or parameter 98a, 98b is used as a selection criterion for use in determining a final decomposed dataset.

In the example of Fig. 7, the method comprises selecting one of the candidate second projection datasets 97a, 97b for use in forming a final decomposed dataset 99 based on the respective value 98a, 98b of said variable or parameter in at least one of the tables. In the particular example of Fig. 7, this selection or decision is effectively performed using the value of the variable or parameter in only one of the tables (e.g. LUT 96b), wherein this value is correlated with predicated SNR of the output of the lookup table 96b (the decomposed data), and wherein if this value is above a certain threshold, a selection signal 95 from LUT 96b indicates to a selection element 99 that the data from LUT 96b should be used, and if below a threshold, a selection signal 95 from LUT 96b indicates to a selection element 99 that the data from LUT 96a should instead be used. In this example, the variables or parameters 98a in the first table 96a might even be omitted. This represents just one specific, and particularly efficient example. In other example, a separate selection/decision module may make a decision, based on the variables or parameters 98a, 98b of both tables.

In the example of Fig. 8, the method comprises forming a weighting combination of at least a subset of the candidate second projection datasets for use in forming a final decomposed dataset 99. The said variables or parameters 98a, 98b are used to set the weighting factors in said weighted sum. In the particular example illustrated in Fig. 8, the weightings are determined based on the variable or parameter 98a in just one of the tables 96b, and wherein the weighting of the other table is set to 1 minus this value, as indicated in Fig. 8. In this way, it can be ensured that all of the weightings sum to one. A weighting selection signal 95 may be output from one of the tables 96b with the weightings. This represents just one particularly efficient example for setting the weightings. In this example, the variables or parameters 98a in the first table 96a might even be omitted. In another example, the variables or parameters 98a, 98b may be output from both of the tables 96a, 96b and sent to a separate weighting determiner which then sets the weightings based on the variables or parameters 98a, 98b so that all weightings sum to one.

The final decomposed data set may be processed with a reconstruction algorithm to form one or more images.

In another alternative example, instead of obtaining the variable or parameter 98a, 98b (the selection criterion ) from the decomposition procedure, it may be obtained based on the candidate decomposed data values 97a, 97b themselves. In some examples, the selection variable or parameter of each candidate decomposed dataset may be a computed signal-to-noise (SNR) ratio estimate associated with the respective candidate decomposed dataset.

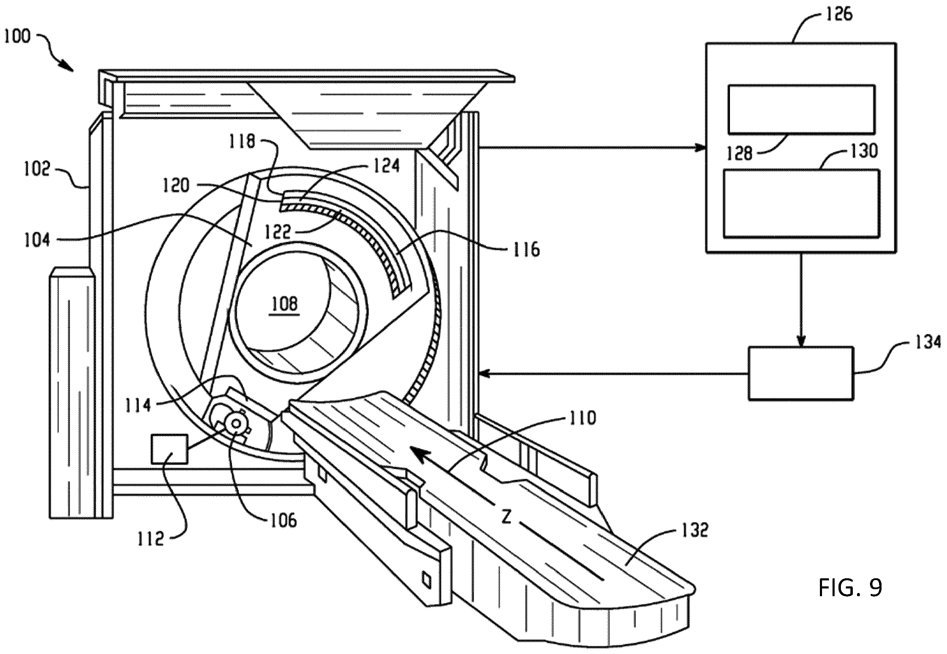

In order to further clarify and give context to above-described embodiments of the invention, Fig. 9 shows an example CT imaging apparatus 100 for acquiring spectral CT imaging data. It is to be noted that the invention does not require the steps of actually acquiring the CT projection data, or of reconstructing the decomposed projection data to form the image data, although these steps can optionally be included as a further part of the method. The following description of Fig. 9 is provided by way of useful background to assist in understanding features comprised by embodiments of the invention. As noted above, in some embodiments, a system may be provided which includes a CT imaging apparatus 100.

With reference to Fig. 9, a computed tomography (CT) scanner 100 includes a generally stationary gantry portion 102 and a rotating gantry portion 104. The rotating gantry portion 104 is rotatably supported by the generally stationary gantry portion 102 via a bearing or the like.

A radiation source 106, such as an x-ray tube, is supported by the rotating gantry portion 104 and rotates therewith around an examination region 108 about a longitudinal or z-axis 110. A source collimator 114 or the like collimates radiation emitted by the radiation source 106, producing a generally cone, fan, wedge or otherwise-shaped radiation beam that traverse the examination region 108.

A radiation source voltage determiner 112 selectively determines the (mean) emission voltage. In one instance, the radiation source voltage determiner 112 switches or changes the emission voltage between successive scans of the same subject/object (this is known as kVp-switching). Alternatively, the radiation source voltage determiner 112 switches the emission voltage during the same scan, for example, from view-to-view, within a view, and/or otherwise. As a result, radiation beams with different energy spectra may be used to scan the subject/object. Since the absorption of photons by a material is dependent on photon energy, the data from the two scans can be used to determine information indicative of the elemental composition, such as an atomic number, of the tissue/material in the scanned subject/object.

In this way, data pertaining to two different spectral channels is obtainable.

By way of non-limiting illustration, the radiation source voltage determiner 112 may switch the emission voltage between about 80 kV and about 140 kV between scans, between views, within a view, and/or otherwise. A filter may be used to filter low energy photons at the higher emission voltage, which may improve the spectral sensitivity of the system. As a consequence of switching emission voltages, the radiation source 106 produces a first radiation beam with a first energy spectrum and a second radiation beam with a second different energy spectrum. Of course, other emission voltages are contemplated, and the radiation source voltage determiner 112 may switch between more than two different emission voltages. An energy-resolving detector array 116 subtends an angular arc opposite the examination region 108 relative to the radiation source 106 and detects radiation that traverses the examination region 108. The illustrated energy-resolving detector array 116 may include a photosensor array 118, with photosensors such as photodiodes or the like, and a scintillator array 120, which is optically coupled to the photosensor array 118 on the light sensitive side of the photosensor array 118. The energy-resolving detector array 116 is arranged in the scanner 100 so that the scintillator 120 receives the incident radiation. Although only a single row energy-resolving detector array 116 is shown, a two dimensional energy-resolving detector array, with rows extending in the z-axis direction and columns extending in a transverse direction, is also contemplated herein.

Alternatively, the detector array may be of the direct conversion type, i.e., comprising a scintillator directly converting incoming radiation into electrical signals.

The illustrated scintillator array 120 includes two or more regions 122, 124 having different spectral sensitivities. The spectral sensitivities of the photosensors in the photosensor array 118 are matched to the emission spectrums of the scintillation regions 122, 124. By way of example, some of the photosensors in the photosensor array 118 detect light emitted by the scintillation region 122 and other photosensors in the photosensor array 118 detect light emitted by the scintillation region 124. Generally, lower energy photons are absorbed in the scintillation region 122 whereas photons that traverse the scintillation region 122 are absorbed in the scintillation region 124.

The energy-resolving detector array 116 outputs a signal or projection data indicative of the detected radiation. The array 116 can be a dual-layer detector array, as energy-sensitive photon counting detector array or other energy-resolving detector array. As the emission voltage may change during a scan and the energy-resolving detector array 116 is sensitive to photon energy, the energy-resolving detector array 116 generates energy-resolved projection data $d_n$, wherein n represents energy-resolved data for the nth energy range. By way of example, in the case where the emission voltage switches between two different emission voltages during a scan and the detector array 116 includes two sets of photosensors with two different spectral sensitivities, the resulting projection data includes four (4) independent energy-resolved measurements (spectral channels), representing the different combinations of two emission voltages and two photosensor spectral sensitivities.

A reconstructor 126 may reconstruct the projection data from the detector array and generates volumetric image data indicative of the examination region 108. As noted above, the reconstructor 126 receives the energy resolved detector signals $d_n$ indicative of the energy detected in n energy ranges. The reconstructor 126 employs one or more spectral decomposition algorithms 128 and/or one or more spectral reconstruction algorithms 130, such as a Maximum Likelihood (ML) reconstruction algorithm that reconstructs spectral data, which take into account the detected spectral information. The resulting reconstructed image data includes a plurality of subsets of image data, each corresponding to a different spectral or material component.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A computed tomography (CT) basis decomposition method, comprising:

    obtaining first projection data comprising, for each of an array of detector pixels, data for each of a first set of three or more spectral channels,
    converting the first projection data to second projection data, wherein the second projection data comprises, for each of the array of detector pixels, data for each of a second set of two or more spectral channels, the second set of spectral channels including at least one virtual spectral channel formed from a combination of two or more of the first set of spectral channels, and wherein the second set comprises fewer channels than the first set;
    applying a basis decomposition procedure to the second projection data to derive basis component data for each of at least two basis components;
    wherein the combination used in the conversion to the second set of spectral channels is determined based on received information about one or more imaging parameters during acquisition of the first projection data, and/or is determined based on analysis of at least a portion of the first projection data.

2. The method of claim 1, wherein the combination used in the conversion to the second set of spectral channels is different for different pixels of the array of detector pixels.

3. The method of claim 1 or 2, wherein the second set of spectral channels includes all of the first set of spectral channels as a single channel or combined in one or more virtual spectral channels.

4. The method of any of claims 1-3, wherein the second set of spectral channels is a set of two spectral channels, and wherein the basis decomposition procedure derives basis component data for a set of two basis components.

5. The method of any of claims 1-4,

    wherein the first spectral CT projection data is data from a dual-layer integrating detector comprising an inner detection layer and outer detection layer, and corresponds to data from a dual-energy kvp-switching CT scan protocol in which x-ray radiation at two or more different energies is intermittently projected toward the detector, whereby each detection layer records measurement data for each of two emitted x-ray energies;
    and wherein the first spectral CT projection data comprises data for four spectral channels, each corresponding to a respective combination of one of the said detection layers of the detector and one of the energy levels of the kvp-switching protocol, whereby the four spectral channels correspond to the following combinations:

Lower kVp energy emission, detected in the inner detection layer (Li),
Lower kVp energy emission, detected in the outer detection layer (Lo),
Higher kVp energy emission, detected in the inner detection layer (Hi), and
Higher kVp energy emission detected in the outer detection layer (Ho).

6. The method of claim 5, wherein the second set of spectral channels comprises a set of two spectral channels, and wherein the second set of spectral channels consists of one of the following combinations:

Li, and a virtual spectral channel comprising a combination of Hi, Lo and Ho;
Hi, and a virtual spectral channel comprising a combination of Li, Lo and Ho;
Lo, and a virtual spectral channel comprising a combination of Li, Hi and Ho;
Ho, and a virtual spectral channel comprising a combination of Li, Hi, and Lo;
a virtual spectral channel comprising a combination of Li and Hi, and a virtual spectral channel comprising a combination of Lo and Ho;
a virtual spectral channel comprising a combination of Li and Lo, and a virtual spectral channel comprising a combination of Hi and Ho;
a virtual spectral channel comprising a combination of Li and Ho, and a virtual spectral channel comprising a combination of Hi and Lo.

7. The method of any of claims 1-6, wherein the set of second spectral channels into which the first projection data is converted is determined individually for each pixel of data, wherein a pixel of data means the first projection data for a single one of the detector pixels.

8. The method of claim 7, wherein the said determining of the second set of spectral channels for each pixel of data is determined based on one or more acquisition parameters or settings of a CT imaging apparatus used to acquire at least the respective pixel of the first projection data.

9. The method of claim 8, wherein the said determining of the second set of spectral channels for each pixel of data is determined based one or more acquisition parameters or settings of a CT imaging apparatus used to acquire the first projection data which vary as a function of time over the course of acquiring a first projection data set, for example, a scanner gantry rotational position, or a patient axial position relative to the scanner gantry, or radiation strength.

10. The method of claim 8 or 9, wherein the determining of the second set of spectral channels for each pixel of data is determined based on radiation strength of the radiation emitted in acquiring at least the respective pixel of the first projection data, and optionally wherein the scan protocol is a dose modulation protocol, wherein the radiation strength of the emitted radiation is varied as a function of time over the course of acquiring a first projection data set.

11. The method of claim 8 or 9, wherein the method comprises:

converting the first projection data into each of a plurality of candidate sets (65a, 65b) of second spectral channels to derive a plurality of candidate second projection data sets;
applying a decomposition procedure (96a, 96b) to each of the candidate second projection data sets to obtain a plurality of candidate decomposed second projection data sets (97a, 97b);
obtaining a variable or parameter (98a, 98b) associated with each candidate decomposed second projection dataset, either from processing of the decomposed data set or from the decomposition procedure;
selecting one of the candidate second projection datasets for use in forming a final decomposed dataset (99) based on the respective values of said variable or parameter, OR, forming a weighted combination of at least a subset of the candidate second projection datasets for use in forming a final decomposed dataset using the said variables or parameters in setting the weighting factors.

12. The method of claim 11,

wherein said variable or parameter of each candidate decomposed dataset is a computed signal-to-noise ratio (SNR) estimate associated with the respective candidate decomposed dataset; or
wherein the decomposition procedure comprises use of a respective lookup table to map each candidate second projection data set to a candidate decomposed second projection data set, and wherein each record of each lookup table corresponding to a particular set of input data values includes a set of decomposed data values and includes said variable or parameter.

13. The method of any of claims 1-12, wherein the method comprises applying a blending operation to the basis component data such as to smooth a transition between basis component data of at least a first pixel and basis component data of at least a second pixel, decomposed using different respective second sets of spectral channels,

14. A computer program product comprising computer program code configured, when executed by a processor, to perform the method of any of claims 1-13.

15. A processing device, comprising

an input/output; and
one or more processors, adapted to:

receive at the input/output first projection data comprising, for each of an array of detector pixels, data for each of a first set of three or more spectral channels,
convert the first projection data to second projection data, wherein the second projection data comprises, for each of the array of detector pixels, data for each of a second set of two or more spectral channels, the second set of spectral channels including at least one virtual spectral channel formed from a combination of two or more of the first set of spectral channels, and wherein the second set comprises fewer channels than the first set;
apply a basis decomposition procedure to the second projection data to derive basis component data for each of at least two basis components;
wherein the second set of spectral channels is determined based on received information about one or more imaging parameters during acquisition of the first projection data, and/or is determined based on analysis of at least a portion of the first imaging data.

```
            ┌─────────────────────────────────┐
            │ Obtain 1st projection data with 1st │ ～ 12
            │       spectral channels         │
            └─────────────────────────────────┘
                            │
                            ▼
            ┌─────────────────────────────────┐
            │ Convert to 2nd projection data with 2nd │ ～ 14
            │       spectral channels         │
            └─────────────────────────────────┘
                            │
                            ▼
            ┌─────────────────────────────────┐
            │      Apply basis decomposition      │ ～ 16
            └─────────────────────────────────┘
```

## FIG. 1

## FIG. 2

**1st Spectral Channels**

```
┌ ─ ─ ─ ─ ─ ─ ─ ─ ─ ─ ─ ┐
            ╱ 62
  ┌─────────────────┐ │ ╲
  │   1st spectral  │
  │   channel 1     │ │
  └─────────────────┘
  ┌─────────────────┐ │
  │   1st spectral  │
  │   channel 2     │ │
  └─────────────────┘
  ┌─────────────────┐ │
  │   1st spectral  │
  │   channel 3     │ │
  └─────────────────┘
  ┌─────────────────┐ │
  │   1st spectral  │
  │   channel 4     │ │
  └─────────────────┘
└ ─ ─ ─ ─ ─ ─ ─ ─ ─ ─ ─ ┘
```

**2nd Spectral Channels**

Combine → Virtual spectral channel 1 (64)

Virtual spectral channel 1 (64)

66

1st spectral channel 4

## FIG. 3

**1st Spectral Channels**

```
┌ ─ ─ ─ ─ ─ ─ ─ ─ ─ ─ ─ ┐
            ╱ 62
  ┌─────────────────┐ │
  │   1st spectral  │
  │   channel 1     │ │
  └─────────────────┘
  ┌─────────────────┐ │
  │   1st spectral  │
  │   channel 2     │ │
  └─────────────────┘
  ┌─────────────────┐ │
  │   1st spectral  │
  │   channel 3     │ │
  └─────────────────┘
  ┌─────────────────┐ │
  │   1st spectral  │
  │   channel 4     │ │
  └─────────────────┘
└ ─ ─ ─ ─ ─ ─ ─ ─ ─ ─ ─ ┘
```

**2nd Spectral Channels**

Combine → Virtual spectral channel 1 (64a)

Virtual spectral channel 1

72

Combine → Virtual spectral channel 2 (64b)

Virtual spectral channel 2

## FIG. 4

FIG. 5

FIG. 6

FIG. 7

FIG. 8

FIG. 9

Europäisches
Patentamt

European
Patent Office

Office européen
des brevets

# EUROPEAN SEARCH REPORT

Application Number

EP 22 18 0728

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/240339 A1 (DU YANFENG [US] ET AL) 2 October 2008 (2008-10-02) | 1-8,14, 15 | INV.<br>A61B6/03 |
| Y | * paragraph [0027]; figures 1,2 * | 9,10 | A61B6/00 |
| A | * paragraph [0042] – paragraph [0046]; figure 7 * | 11-13 | G01T1/20<br>G06T11/00 |
|  | ----- |  |  |
| Y | US 2020/085402 A1 (OBLER RICHARD [DE] ET AL) 19 March 2020 (2020-03-19) | 9,10 |  |
| A | * paragraphs [0050], [0079]; figure 3 * | 1-8, 11-15 |  |
|  | ----- |  |  |

|  |
|---|
| TECHNICAL FIELDS<br>SEARCHED (IPC) |
| A61B<br>G01V<br>G06T<br>G01T |

The present search report has been drawn up for all claims

| Place of search | Date of completion of the search | Examiner |
|---|---|---|
| Munich | 12 September 2022 | Marzal-Abarca, X |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

....................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 22 18 0728**

This annex lists the patent family members relating to the patent documents cited in the above-mentioned European search report.
The members are as contained in the European Patent Office EDP file on
The European Patent Office is in no way liable for these particulars which are merely given for the purpose of information.

**12-09-2022**

| Patent document cited in search report | Publication date | Patent family member(s) | Publication date |
|---|---|---|---|
| US 2008240339 A1 | 02-10-2008 | NONE | |
| US 2020085402 A1 | 19-03-2020 | CN 110916692 A | 27-03-2020 |
| | | DE 102018215958 A1 | 19-03-2020 |
| | | US 2020085402 A1 | 19-03-2020 |

EPO FORM P0459

For more details about this annex : see Official Journal of the European Patent Office, No. 12/82